(19) Europäisches Patentamt
European Patent Office
Office européen des brevets

(11) Veröffentlichungsnummer: **0 072 989**
**A1**

(12) # EUROPÄISCHE PATENTANMELDUNG

(21) Anmeldenummer: 82107365.7

(22) Anmeldetag: 13.08.82

(51) Int. Cl.[3]: C 07 J 71/00
A 61 K 31/58

(30) Priorität: 21.08.81 DE 3133631

(43) Veröffentlichungstag der Anmeldung: 02.03.83 Patentblatt 83/9

(84) Benannte Vertragsstaaten: AT BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Annen, Klaus, Dr.
Seegefelder Strasse 194
D-1000 Berlin 20(DE)

(72) Erfinder: Laurent, Henry, Dr.
Glambecker Weg 21
D-1000 Berlin 28(DE)

(72) Erfinder: Hofmeister, Helmut, Dr.
Weislingenstrasse 4
D-1000 Berlin 28(DE)

(72) Erfinder: Wendt, Hans, Dr.
Ernst-Ring-Strasse 14
D-1000 Berlin 38(DE)

(72) Erfinder: Albring, Manfred, Dr.
Im Ellenbügel 81
D-6456 Langenselbold(DE)

(54) **Neue Kortikoide ihre Herstellung und Verwendung.**

(57) Kortikoide der allgemeinen Formel I

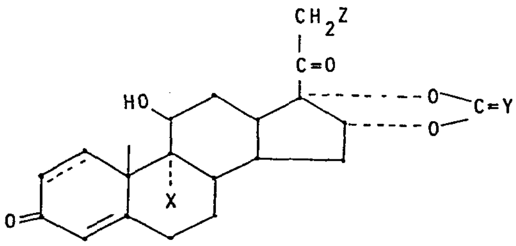

(1),

worin
⋮ eine Einfachbindung oder eine Doppelbindung,
X ein Wasserstoffatom, ein Fluoratom oder ein Chloratom,
Y zwei Wasserstoffatome oder ein Sauerstoffatom und
Z ein Wasserstoffatom oder einen Acyloxyrest mit 2 bis 6 Kohlenstoffatomen darstellen, sind pharmakologisch wirksame Substanzen.

EP 0 072 989 A1

Croydon Printing Company Ltd.

Die Erfindung betrifft den in den Patentansprüchen gekennzeichneten Gegenstand.

Die neuen Kortikoide der allgemeinen Formel I gemäß Patentanspruch 1 können als 2 bis 6 Kohlenstoffatome enthaltende Acyloxygruppen Z beispielsweise eine Acetoxygruppe, eine Propionyloxygruppe, eine Butyryloxygruppe, eine Isobutyryloxygruppe, eine Valeryloxygruppe, eine 3-Methylbutyryloxygruppe, eine Trimethylacetoxygruppe oder eine Hexanoyloxygruppe tragen.

Die im Anspruch 1 gekennzeichneten Kortikoide besitzen bei topischer Applikation eine stark antiinflammatorische Wirksamkeit und sind bei systemischer Applikation relativ schwach wirksam.

Die neuen Kortikoide der allgemeinen Formel I gemäß Patentanspruch 1 eigenen sich demzufolge in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen Pruritis vulvae et ani, Rosacea, Erythematodes cutaneus, Psoriasis, Lichen ruber planus et verrucosus und ähnlichen Hauterkrankungen.

Die Herstellung der Arzneimnittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die neuen Kortikoide können nach den in dem Patentanspruch 23 gekennzeichneten Verfahren hergestellt werden.

Die nachfolgenden Ausführungsbeispiele dienen zur Erläuterung des erfindungsgemäßen Verfahrens.

Beispiel 1

a) Zu einer Lösung von 34.4 g 21-Acetoxy-16α,17α-dihydroxy-1,4,9-pregnatrien-3,20-dion in 254 ml wasserfreiem Methylenchlorid und 164 ml wasserfreiem Formaldehyddimethylacetal wird unter Rühren bei Raumtemperatur portionsweise ein Gemisch aus 54.6 g Kieselgur W20 und 27.3 g Phosphorpentoxid hinzugefügt. Man rührt 1 Stunde nach, saugt ab, wäscht den Rückstand mit triäthylaminhaltigem Methylenchlorid und engt das Filtrat i.Vak. zur Trockne ein. Man reinigt das Rohprodukt an 3.5 kg Kieselgel mit einem Methylenchlorid-Aceton-Gradienten (0-12 % Aceton). Ausbeute: 29.7 g 21-Acetoxy-16α,17α-methylendioxy-1,4,9-pregnatrien-3,20-dion. Schmelzpunkt 200°C.

b) Eine Lösung von 12.0 g 21-Acetoxy-16α,17α-methylendioxy-1,4,9-pregnatrien-3,20-dion in 120 ml Dioxan wird bei Raumtemperatur unter Rühren mit 11.28 g N-Bromsuccinimid versetzt. Man tropft 60 ml einer 10proz. wäßrigen Perchlorsäure-Lösung hinzu, rührt 10 Minuten bei Raumtemperatur weiter und gibt auf eine Eiswasser-Kochsalz-Lösung. Nach der üblichen Aufarbeitung erhält man 16.6 g 21-Acetoxy-9α-brom-11ß-hydroxy-16α,17α-methylendioxy-1,4-pregnadien-3,20-dion, die aus Aceton/Hexan umkristallisiert werden. Schmelzpunkt 188°C.

c) Man refluxiert eine Lösung von 10.2 g 21-Acetoxy-9α-brom-11ß-hydroxy-16α,17α-methylendioxy-1,4-pregnadien-3,20-dion in 210 ml Tetrahydrofuran mit 72.25 ml Tri-n-butylzinnhydrid und einer Spatelspitze Azobisisobutyronitril 1 Stunde lang. Nachdem man die Reaktionslösung i.Vak. zur Trockne eingeengt hat, reinigt man den Rückstand an 1.1 kg Kieselgel, indem man zunächst mit 5 l Hexan und anschließend mit einem Methylenchlorid-Aceton-Gradienten (0-15 % Aceton) eluiert. Ausbeute: 7.9 g 21-Acetoxy-11ß-hydroxy-16α,17α-methylendioxy-1,4-pregnadien-3,20-dion. Schmelzpunkt 271°C (Zers.).

Beispiel 2

1.5 g 21-Acetoxy-11ß-hydroxy-16α,17α-methylendioxy-1,4-pregnadien-3,20-dion werden in 510 ml Methanol und 111 ml Wasser gelöst und mit 1.5 g Calciumcarbonat 24 Stunden unter Rühren refluxiert. Man saugt das Calciumcarbonat ab, engt das Filtrat bis zur Trübung ein und gibt auf Wasser. Nach der üblichen Aufarbeitung reinigt man das Rohprodukt an 115 g Kieselgel mit einem Methylenchlorid-Aceton-Gradienten (0-20 % Aceton). Man isoliert 758 mg 11ß,21-Dihydroxy-16α,17α-methylendioxy-1,4-pregnadien-3,20-dion. Schmelzpunkt 220-223°C (Zers.).

Beispiel 3

Eine Lösung von 2.4 g 11ß,21-Dihydroxy-16α,17α-methylendioxy-1,4-pregnadien-3,20-dion in 24 ml Pyridin und 12 ml Propionsäureanhydrid wird 1 Stunde bei Raumtemperatur gerührt. Nach der Eiswasser-Kochsalz-Fällung wird abfiltriert und wie üblich aufgearbeitet. Das Rohprodukt wird an 220 g Kieselgel mit einem Methylenchlorid-Aceton-Gradienten (0-12 % Aceton) gereinigt. Man erhält 2.5 g 11ß-Hydroxy-16α,17α-methylendioxy-21-propionyloxy-1,4-pregnadien-3,20-dion. Schmelzpunkt 184-185°C.

Beispiel 4

2.4 g 11ß,21-Dihydroxy-16α,17α-methylendioxy-1,4-pregnadien-3,20-dion werden analog Beispiel 3 mit Buttersäureanhydrid umgesetzt, aufgearbeitet und gereinigt. Man isoliert 2.6 g 21-Butyryloxy-11ß-hydroxy-16α,17α-methylendioxy-1,4-pregnadien-3,20-dion. Schmelzpunkt 140-141°C.

0072989

Beispiel 5

Analog Beispiel 3 werden 2.2 g 11ß,21-Dihydroxy-16α,17α-methylendioxy-1,4-pregnadien-3,20-dion mit n-Valeriansäureanhydrid umgesetzt, aufgearbeitet und gereinigt. Man erhält 2.1 g 11ß-Hydroxy-16α,17α-methylendioxy-21-valeryloxy-1,4-pregnadien-3,20-dion. Schmelzpunkt 121-122°C.

Beispiel 6

Eine Suspension von 4.2 g Tristriphenylphosphin-rhodium-I-chlorid in 195 ml Benzol und 65 ml Methanol wird 1 Stunde bei Raumtemperatur unter Normaldruck vorhydriert. Man fügt anschließend 5.2 g 21-Acetoxy-11ß-hydroxy-16α,17α-methylen-dioxy-1,4-pregnadien-3,20-dion in 195 ml Benzol und 65 ml Methanol hinzu und hydriert 5 Stunden unter Normaldruck weiter. Die Reaktionslösung wird i.Vak. zur Trockne eingeengt und der Rückstand an 1 kg Kieselgel mit einem Hexan-Essigester-Gradienten (0-60 % Essigester) gereinigt. Ausbeute: 3.8 g 21-Acetoxy-11ß-hydroxy-16α,17α-methylendioxy-4-pregnen-3,20-dion. Schmelzpunkt 241-243°C.

Beispiel 7

a) Eine Suspension von 3.0 g 21-Acetoxy-9α-brom-11ß-hydroxy-16α,17α-methylendioxy-1,4-pregnadien-3,20-dion in 180 ml Äthanol wird mit 4.2 g Kaliumacetat 2 1/2 Stunden refluxiert. Man engt ein und gibt das konzentrierte Reaktionsgemisch auf eine Eiswasser-Kochsalz-Lösung. Nach dem Abfiltrieren und Waschen des Rückstandes arbeitet man wie üblich auf und reinigt das Rohprodukt an 220 g Kieselgel mit einem Methylenchlorid-Aceton-Gradienten (0-15 % Aceton). Ausbeute: 1.83 g 21-Acetoxy-9ß,11ß-epoxy-16α,17α-methylendioxy-1,4-pregnadien-3,20-dion. Schmelzpunkt 168-169°C.

b) 1.5 ml einer 70proz. HF/Pyridin-Lösung werden auf -35°C abgekühlt und mit 400 mg 21-Acetoxy-9ß,11ß-epoxy-16α,17α-methylendioxy-1,4-pregnadien-3,20-dion versetzt. Man rührt das Reaktionsgemisch 8 Stunden bei -5°C und gibt anschließend auf ammoniakalisches Eiswasser. Man filtriert ab, wäscht den Rückstand neutral und arbeitet wie üblich auf. Das Rohprodukt wird an 65 g Kieselgel mit einem Methylenchlorid-Aceton-Gradienten (0-15 % Aceton) gereinigt. Es werden 377 mg 21-Acetoxy-9α-fluor-11ß-hydroxy-16α,17α-methylendioxy-1,4-pregnadien-3,20-dion isoliert. Schmelzpunkt 283-285°C.

Beispiel 8

18.0 g 21-Acetoxy-9α-fluor-11ß-hydroxy-16α,17α-methylendioxy-1,4-pregnadien-3,20-dion werden in einem Gemisch aus 216 ml 60proz. Perchlorsäure und 720 ml Methanol bei Raumtemperatur 18 Stunden gerührt. Die Reaktionslösung wird mit Methylenchlorid verdünnt und auf Wasser gegeben. Man extrahiert mit Methylenchlorid und erhält nach dem Neutralisieren, Waschen, Trocknen und Einengen der organischen Extrakte 14.0 g Rohprodukt, das aus Hexan/Aceton umkristallisiert wird und 10.6 g 9α-Fluor-11ß,21-dihydroxy-16α,17α-methylendioxy-1,4-pregnadien-3,20-dion liefert. Schmelzpunkt 262°C.

Beispiel 9

a) Eine Lösung von 2.0 g 9α-Fluor-11ß,21-dihydroxy-16α,17α-methylendioxy-1,4-pregnadien-3,20-dion in 20 ml Pyridin wird 1 Stunde bei Raumtemperatur mit 2.4 g Tosylchlorid gerührt und auf Eiswasser-Kochsalz-Lösung gegeben. Nach der üblichen Aufarbeitung isoliert man 2.4 g 9α-Fluor-11ß-hydroxy-16α,17α-methylendioxy-21-tosyloxy-1,4-pregnadien-3,20-dion.

b) 2.4 g 9α-Fluor-11ß-hydroxy-16α, 17α-methylendioxy-21-tosyloxy-1,4-pregnadien-3,20-dion werden in 48 ml Hexamethylphosphorsäuretriamid suspendiert und mit 2.4 g Lithiumchlorid 2 1/2 Stunden bei 80°C gerührt. Man gibt auf eine Eiswasser-Kochsalz-Lösung, filtriert ab und arbeitet den Rückstand wie üblich auf. Das Rohprodukt wird an 190 g Kieselgel mit einem Methylenchlorid-Aceton-Gradienten (0-15 % Aceton) gereinigt. Ausbeute: 1.2 g 21-Chlor-9α-fluor-11ß-hydroxy-16α, 17α-methylendioxy-1,4-pregnadien-3,20-dion. Schmelzpunkt 264-265°C.

Beispiel 10

Unter den Bedingungen des Beispiels 6 werden 3.2 g 21-Acetoxy-9α-fluor-11ß-hydroxy-16α, 17α-methylendioxy-1,4-pregnadien-3,20-dion hydriert und aufgearbeitet. Die Reinigung erfolgt an 350 g Kieselgel mit einem Hexan-Essigester-Gradienten (0-80 % Essigester). Man isoliert 2.6 g 21-Acetoxy-9α-fluor-11ß-hydroxy-16α, 17α-methylendioxy-4-pregnen-3,20-dion. Schmelzpunkt 264-266°C.

Beispiel 11

Man versetzt eine Lösung von 2.0 g 21-Acetoxy-16α, 17α-methylendioxy-1,4,9-pregnatrien-3,20-dion in 20 ml Dioxan mit 1.88 g N-Chlorsuccinimid und tropft unter Rühren 10 ml einer 10proz. wäßrigen Perchlorsäurelösung hinzu. Die Reaktionslösung wird 2 1/2 Stunden bei Raumtemperatur gerührt und man gibt anschließend auf eine Eiswasser-Kochsalz-Lösung. Der Niederschlag wird abfiltriert, gewaschen und wie üblich aufgearbeitet. Das Rohprodukt wird an 200 g Kieselgel mit einem Methylenchlorid-Aceton-Gradienten (0-15 % Aceton) gereinigt. Ausbeute: 1.39 g 21-Acetoxy-9α-chlor-11ß-hydroxy-16α, 17α-methylendioxy-1,4-pregnadien-3,20-dion. Schmelzpunkt 244-245°C.

Beispiel 12

a) Analog Beispiel 6 werden 6.0 g 21-Acetoxy-16α,17α-methylendioxy-1,4,9-pregnatrien-3,20-dion über Tristriphenylphosphin-rhodium-I-chlorid hydriert, aufgearbeitet und gereinigt. Es werden 5.4 g 21-Acetoxy-16α,17α-methylendioxy-4,9-pregnadien-3,20-dion isoliert. Schmelzpunkt 201-202°C.

b) Unter den Bedingungen des Beispiels 11 werden 2.0 g 21-Acetoxy-16α,17α-methylendioxy-4,9-pregnadien-3,20-dion mit N-Chlorsuccinimid umgesetzt, aufgearbeitet und gereinigt. Man isoliert 920 mg 21-Acetoxy-9α-chlor-11ß-hydroxy-16α,17α-methylendioxy-4-pregnen-3,20-dion. Schmelzpunkt 231°C.

Beispiel 13

Bei 0°C wird eine Lösung von 6.0 g 21-Acetoxy-11ß,16α,17α-trihydroxy-1,4-pregnadien-3,20-dion in 60 ml Pyridin tropfenweise mit 4.5 ml Chlorameisensäureäthylester versetzt und 30 Minuten weitergerührt. Die Reaktionslösung wird auf eine Eiswasser-Kochsalz-Lösung gegeben, der Niederschlag abfiltriert und nach dem Waschen wie üblich aufgearbeitet. Das Rohprodukt wird an 750 g Kieselgel mit einem Methylenchlorid-Aceton-Gradienten (0-20 % Aceton) gereinigt. Ausbeute: 4.76 g 21-Acetoxy-11ß-hydroxy-1,4-pregnadienø-[16α,17α-d]-[1,3]-dioxolan-2',3,20-trion. Schmelzpunkt 250-251°C.

Beispiel 14

Unter den Bedingungen des Beispiels 6 werden 1.5 g 21-Acetoxy-11ß-hydroxy-1,4-pregnadieno-[16α,17α-d]-[1,3]-dioxolan-2',3,20-trion hydriert und aufgearbeitet. Das Rohprodukt wird an 200 g Kieselgel mit einem Methylenchlorid-Aceton-Gradienten (0-15 % Aceton) gereinigt. Man isoliert 1.1 g 21-Acetoxy-11ß-hydroxy-4-pregneno-[16α,17α-d]-[1,3]-dioxolan-2',3,20-trion. Schmelzpunkt 232-234°C.

0072989

Beispiel 15

a) 2.5 g 21-Acetoxy-16α,17α-dihydroxy-1,4,9-pregnatrien-3,20-dion in 25 ml Pyridin werden mit 1.9 ml Chlorameisensäureäthylester analog Beispiel 13 umgesetzt, aufgearbeitet und gereinigt. Ausbeute: 1.93 g 21-Acetoxy-1,4,9-pregnatrieno-[16α,17α-d]-[1,3]-dioxolan-2',3,20-trion. Schmelzpunkt 204-206°C.

b) 1.2 g 21-Acetoxy-1,4,9-pregnatrieno-[16α,17α-d]-[1,3]-dioxolan-2',3,20-trion werden analog Beispiel 11 mit N-Chlorsuccinimid umgesetzt, aufgearbeitet und gereinigt. Man erhält 595 mg 21-Acetoxy-9α-chlor-11ß-hydroxy-1,4-pregnadieno-[16α,17α-d]-[1,3]-dioxolan-2',3,20-trion. Schmelzpunkt 247-248°C.

Beispiel 16

1.0 g 21-Acetoxy-9α-chlor-11ß-hydroxy-1,4-pregnadieno-[16α,17α-d]-[1,3]-dioxolan-2',3,20-trion werden in 50 ml Methylenchlorid und 850 ml Methanol mit einem Gemisch aus 100 ml Wasser und 200 ml konz. Salzsäure versetzt und 15 Stunden bei Raumtemperatur gerührt. Man neutralisiert die Reaktionslösung mit Sodalösung, engt ein und gibt auf Eiswasser. Man isoliert 500 mg 9α-Chlor-11ß,21-dihydroxy-1,4-pregnadieno-[16α,17α-d]-[1,3]-dioxolan-2',3,20-trion, das aus Hexan/Aceton kristallisiert. Schmelzpunkt 300°C.

Beispiel 17

a) Analog Beispiel 6 werden 6.0 g 21-Acetoxy-1,4,9-pregnatrieno-[16α,17α-d]-[1,3]-dioxolan-2',3,20-trion hydriert, aufgearbeitet und gereinigt. Man isoliert 5.1 g 21-Acetoxy-4,9-pregnadieno-[16α,17α-d]-[1,3]-dioxolan-2',3,20-trion. Schmelzpunkt 221-223°C.

b) Unter den Bedingungen des Beispiels 12 werden 2.0 g 21-Acetoxy-4,9-pregnadieno-[16α,17α-d]-[1,3]-dioxolan-2',3,20-trion mit 1.9 g N-Chlorsuccinimid umgesetzt, aufgearbeitet und gereinigt. Ausbeute: 700 mg 21-Acetoxy-9α-chlor-11ß-hydroxy-4-pregneno-[16α,17α-d]-[1,3]-dioxolan-2',3,20-trion. Schmelzpunkt 217-218°C.

Beispiel 18

a) Eine Suspension von 6.0 g 16α,17α-Dihydroxy-21-propionyloxy-4,9-pregnadien-3,20-dion in Pyridin wird analog Beispiel 15a mit Chlorameisensäureäthylester umgesetzt, aufgearbeitet und gereinigt. Man isoliert 5.5 g 21-Propionyloxy-4,9-pregnadieno-[16α,17α-d]-[1,3]-dioxolan-2',3,20-trion vom Schmelzpunkt 177-178°C.

b) 3.2 g 21-Propionyloxy-4,9-pregnadieno-[16α,17α-d]-[1,3]-dioxolan-2',3,20-trion werden analog Beispiel 12 mit N-Chlorsuccinimid umgesetzt, aufgearbeitet und chromatographiert. Ausbeute: 1.62 g 9α-Chlor-11ß-hydroxy-21-propionyloxy-4-pregneno-[16α,17α-d]-[1,3]-dioxolan-2',3,20-trion. Schmelzpunkt 222-223°C.

Beispiel 19

a) 8.6 g 21-Butyryloxy-16α,17α-dihydroxy-4,9-pregnadien-3,20-dion in 100 ml Pyridin werden unter den Bedingungen des Beispiels 15a mit 5.6 ml Chlorameisensäureäthylester umgesetzt, aufgearbeitet und gereinigt. Man isoliert 7.9 g 21-Butyryloxy-4,9-pregnadieno-[16α,17α-d]-[1,3]-dioxolan-2',3,20-trion vom Schmelzpunkt 178-179°C.

b) 7.5 g 21-Butyryloxy-4,9-pregnadieno-[16α,17α-d]-[1,3]-dioxolan-2',3,20-trion werden, wie im Beispiel 12 beschrieben, mit 7.0 g N-Chlorsuccinimid umgesetzt, aufgearbeitet und gereinigt. Man erhält 3.1 g 21-Butyryloxy-9α-chlor-11ß-hydroxy-4-pregneno-[16α,17α-d]-[1,3]-dioxolan-2',3,20-trion. Schmelzpunkt 241-242°C.

Beispiel 20

Unter den Bedingungen des Beispiels 13 werden 1.5 g 21-Acetoxy-9α-fluor-11ß,16α,17α-trihydroxy-1,4-pregnadien-3,20-dion mit Chlorameisensäureäthylester umgesetzt und aufgearbeitet. Man reinigt das Rohprodukt durch Kristallisation aus Aceton/Hexan. Ausbeute: 1.1 g 21-Acetocy-9α-fluor-11ß-hydroxy-1,4-pregnadieno-[16α,17α-d]-[1,3]-dioxolan-2',3,20-trion. Schmelzpunkt 204-205°C.

Patentansprüche

1. Kortikoide der allgemeinen Formel I

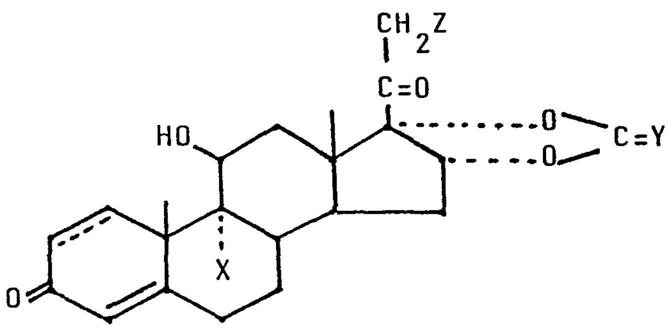

(I),

worin

..... eine Einfachbindung oder eine Doppelbindung,

X ein Wasserstoffatom, ein Fluoratom oder ein Chloratom,

Y zwei Wasserstoffatome oder ein Sauerstoffatom und

Z ein Wasserstoffatom oder einen Acyloxyrest mit 2 bis 6 Kohlenstoffatomen darstellen.

2. 21-Acetoxy-11ß-hydroxy-16α,17α-methylendioxy-1,4-pregnadien-3,20-dion.

3. 11ß,21-Dihydroxy-16α,17α-methylendioxy-1,4-pregnadien-3,20-dion.

4. 21-Butyryloxy-11ß-hydroxy-16α,17α-methylendioxy-1,4-pregnadien-3,20-dion.

5. 11ß-Hydroxy-16α,17α-methylendioxy-21-valeryloxy-1,4-pregandien-3,20-dion.

6. 9α-Fluor-11β,21-dihydroxy-16α,17α-methylendioxy-1,4-pregnadien-3,20-dion.

7. 21-Chlor-9α-fluor-11β-hydroxy-16α,17α-methylendioxy-1,4-pregnadien-3,20-dion.

8. 21-Acetoxy-11β-hydroxy-1,4-pregnadieno[16α,17α-d]-[1,3]-dioxolan-2',3,20-trion.

9. 21-Acetoxy-11β-hydroxy-16α,17α-methylendioxy-4-pregnen-3,20-dion.

10. 11β-Hydroxy-16α,17α-methylendioxy-21-propionyloxy-1,4-pregnadien-3,20-dion.

11. 21-Acetoxy-9α-chlor-11β-hydroxy-16α,17α-methylendioxy-1,4-pregnadien-3,20-dion.

12. 21-Acetoxy-9α-fluor-11β-hydroxy-16α,17α-methylendioxy-1,4-pregnadien-3,20-dion.

13. 21-Acetoxy-9α-fluor-11β-hydroxy-16α,17α-methylendioxy-4-pregnen-3,20-dion.

14. 21-Acetoxy-11β-hydroxy-4-pregneno-[16α,17α-d]-[1,3]dioxolan-2',3,20-trion.

15. 21-Acetoxy-9α-chlor-11β-hydroxy-1,4-pregnadieno-[16α,17α-d]-[1,3]dioxolan-2',3,20-trion.

16. 9α-Chlor-11β,21-dihydroxy-1,4-pregenadieno-[16α,17α-d]-[1,3]dioxolan-2',3,20-trion.

17. 21-Acetoxy-9α-chlor-11β-hydroxy-4-pregneno-[16α,17α-d]-[1,3]dioxolan-2',3,20-trion.

18. 9α-Chlor-11ß-hydroxy-21-propionyloxy-4-pregneno[16α,17α-d]-[1,3]dioxolan-2',3,20-trion.

19. 21-Butyryloxy-9α-chlor-11ß-hydroxy-4-pregneno-[16α,17α-d]-[1,3]dioxolan-2',3,20-trion.

20. 21-Acetoxy-9α-fluor-11ß-hydroxy-1,4-pregnadieno-[16α,17α-d]-[1,3]dioxolan-2',3,20-trion.

21. 21-Acetoxy-9α-chlor-11ß-hydroxy-16α,17α-methylendioxy-4-pregnen-3,20-dion.

22. Pharmazeutische Präparate gekennzeichnet durch den Gehalt an ein oder zwei Kortikoiden gemäß Anspruch 1 bis 21.

23. Verfahren zur Herstellung von Kortikoiden der allgemeinen Formel I

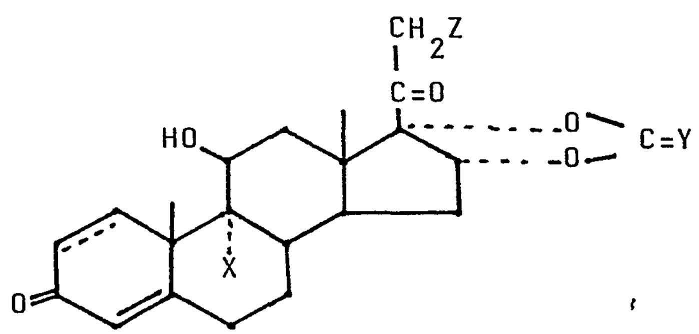

(I),

worin

..... eine Einfachbindung oder eine Doppelbindung,
X ein Wasserstoffatom, ein Fluoratom oder ein Chloratom,
Y zwei Wasserstoffatome oder ein Sauerstoffatom und
Z ein Wasserstoffatom oder einen Acyloxyrest mit 2 bis 6 Kohlenstoffatomen darstellen,dadurch gekennzeichnet, daß man

a) ein Kortikoid der allgemeinen Formel II

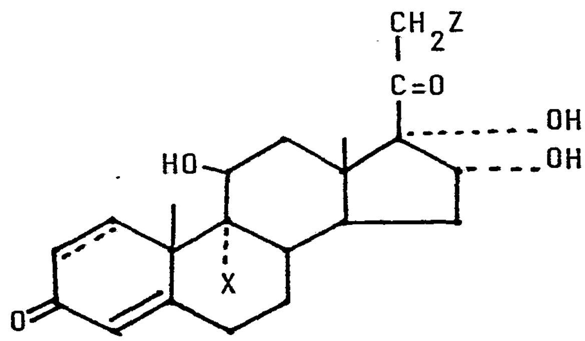

(II),

worin

....., X und Z die obengenannte Bedeutung besitzen, mit einer Verbindung der allgemeinen Formel III

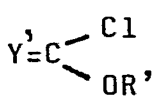

(III),

worin

Y ein Sauerstoffatom und R' jeweils einen 1 bis 4 Kohlenstoffatome enthaltender Alkylrest darstellt, kondensiert, oder

b) an die $\Delta^{9(11)}$-Doppelbindung eines Kortikoids der allgemeinen Formel IV

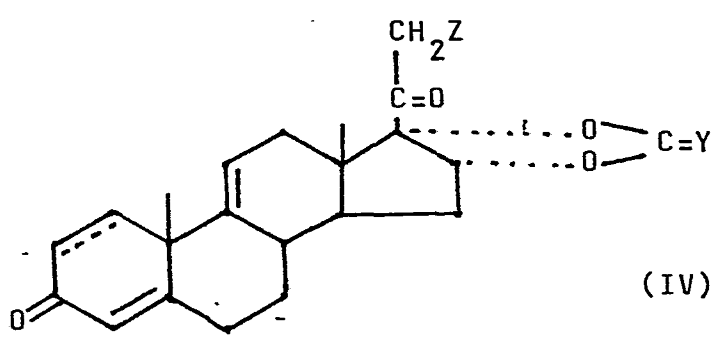

(IV),

worin

....., Y und Z die obengenannte Bedeutung besitzen unterchlorige oder unterbromige Säure anlagert, gegebenenfalls das 9-Halogenatom eliminiert oder die 11ß-Hydroxy-9-halo-

genverbindung in das 9,11ß-Epoxid überführt und dieses mit Fluorwasserstoff oder Chlorwasserstoff öffnet, gewünschtenfalls die gemäß Verfahrensvariante a oder b erhaltenen $\Delta^{1,4}$-Kortikoide der allgemeinen Formel I zu den entsprechenden $\Delta^{4}$-Kortikoiden hydriert, 21-Acetylverbindungen mit Y in der Bedeutung zweier Wasserstoffatome zu den entsprechenden 21-Hydroxyverbindungen verseift und 21-Hydroxysteroide der allgemeinen Formel I verestert oder in die 21-Chlorverbindungen überführt.

Europäisches Patentamt

# EUROPÄISCHER RECHERCHENBERICHT

0072989

Nummer der Anmeldung

EP 82 10 7365

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US-A-3 069 420 (JOSEF FRIED) * Ansprüche 1,3-5; Spalten 1-4 * | 1,8,14-20,22 | C 07 J 71/00<br>A 61 K 31/58 |
| Y | GB-A- 909 126 (AMERICAN CYANAMID) * Ansprüche 1,2; Seite 1 * | 1,22 | |
| Y | DE-B-1 131 213 (SYNTEX) * Beispiele 1,2; Spalte 1 * | 1,22 | |
| Y | CHEMICAL ABSTRACTS, Band 94, Nr. 3, 19. Januar 1981, Seite 472, Nr. 15993r, Columbus, Ohio, USA & ES - A - 483 720 (COMPANIA ESPANOLA DE ESTEROIDES S.A.) 16.05.1980 * Zusammenfassung * | 1,22 | |
| A | US-A-3 050 519 (JOSEF FRIED) | | |

RECHERCHIERTE SACHGEBIETE (Int. Cl. 3)

C 07 J 71/00

Der vorliegende Recherchenbericht wurde für alle Patentansprüche erstellt.

| Recherchenort | Abschlußdatum der Recherche | Prüfer |
|---|---|---|
| DEN HAAG | 04-11-1982 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82